# EUROPEAN PATENT APPLICATION

(11) **EP 4 056 223 A1**
(43) Date of publication of application: **14.09.2022**
(21) Application number: 21177803.0
(22) Date of filing: 04.06.2021
(51) Int. Cl.: A61M 37/00

(54) **DEVICE FOR AUTOMATICALLY OPERATING STAMP-TYPE FLUID INJECTOR HAVING FINE NEEDLES FOR HAIR LOSS CARE AND TREATMENT**

(30) Priority: 09.03.2021 KR 20210030701
(71) Applicant: Han, Sangbum, Uichang-gu Changwon-si Gyeongsangnam-do (KR)
(72) Inventor: Han, Sangbum, Uichang-gu Changwon-si Gyeongsangnam-do (KR)
(74) Representative: Cabinet Chaillot

(57) **Abstract**

A device for automatically operating a stamp-type fluid injector 200 having fine needles 256 for hair loss care and treatment, the device caring and treating hair loss by supplying a liquid medicine while making injuries on the scalp using fine needles. In particular, a hole 22 is formed at the center of a cover 20 coupled to the lower portion of an outer case 10 such that an outer cap 40 is moved up and down in the hole by a motor M. An inner cap 70 elastically operated by a spring 60 is disposed under the outer cap, a flexible cap is disposed in the inner cap, and a fluid injector is simply inserted into and separated out of the flexible cap through the hole of the cover.

## Description

### BACKGROUND OF THE INVENTION

### Field of the Invention

The preset disclosure relates to a device for automatically operating a stamp-type fluid injector having fine needles for hair loss care and treatment. More particularly, the present disclosure relates to a device for automatically operating a stamp-type fluid injector having fine needles for hair loss care, the device: making fine injuries by tapping the fine needles of the fluid injector on the scalp so that hair loss is cared and treated well by production of capillaries, reproduction of cells, and permeation of a liquid medicine even into hair roots during recovery of the injuries; having the fluid injector simply mounted and separated; and being able to continuously and uniformly tap the scalp while automatically moving up and down the fluid injector.

### Description of the Related Art

Human loose some hair daily, but hair is continuously replaced, so the amount of hair is always maintained.

The hair cycle can be divided into three phases of anagen, catagen, and talogen. Anagen is a phase in which cell division actively occurs at dermal papillae and growth of new hairs is promoted, and hairs can grow in this phase.

The growth cycle of hairs is about 3 to 5 years for men and 4 to 6 years for women and about 80 to 85% hairs of the entire hairs are in anagen.

Catagen, which is a period in which cell disorganization gradually stops, is about 3 to 4 weeks and talogen, which is a period in which dermal papillae contract and hairs are separated from capillaries and are merely stuck in the scalp, is about 3 months, and hairs are easily separated from the scalp by physical stimulus in talogen.

Recently, the hair loss population is rapidly increasing due to excessive stress by advancement and industrialization, lifestyle, and other various factors. Hair loss is increasing not only for middle-aged men, but for younger population, so studies for analyzing and removing various factors of hair loss are being actively conducted.

One of various methods for solving hair loss is a method of supplying nutrition or liquid treatment medicines to the scalp.

Such liquid medicines are provided together with various components in a container to be supplied to the scalp by an applicator.

However, since existing applicators are used to simply spray and apply a liquid medicine to the scalp, there is a problem that the liquid medicine cannot permeate well even to the hair roots and it difficult to achieve a satisfactory effect in terms of hair care and treatment.

In order to solve this problem, a stamp-type apparatus 200 for injecting fluid has been registered in Korean Patent No. 10-1993706. The stamp-type apparatus, as shown in FIGS. 12 and 13, is configured to be coupled to a liquid medicine capsule 210 and includes a connection housing 230, a cap 240, and a needle assembly 250. The connection housing 230 is coupled to the liquid medicine capsule and has a nozzle hole 232 having a tapered upper end at the center thereof. The needle assembly 250 is coupled to the lower portion of the connection housing and has several needle plates 255 having fine needles 256 protruding from a body 251. The cap 240 has a top pin fitted in the nozzle hole 232 of the connection housing and a bottom pin 242 protruding downward from the needle assembly, in which the top pin 241 and the bottom pin 242 are coupled up and down and inserted in a spring 245 to elastically move up and down. When a user taps the bottom of the stamp-type apparatus 200 on the scalp, the fine needles make fine injuries on the scalp and the cap elastically moves up, whereby the nozzle hole 232 is opened and the liquid medicine is supplied. Accordingly, there is an effect that capillaries are produced in the process of recovering from the injuries after the injuries are made by the fine needles, so blood circulation is improved and the liquid medicine effectively permeates even to the hair roots with reproduction of cells, whereby the effect of hair loss care and treatment is increased.

However, since a user has to hold the stamp-type apparatus 200 with a hand and repeatedly tap the apparatus on the scalp to use it, it is very difficult for a user to taps on the entire scalp and it is difficult to use the stamp-type apparatus while uniformly tapping the scalp.

In particular, when the force that is applied to the scalp of the stamp-type apparatus tapping the scalp is small, fine injuries are not made on the scalp, so the effect of production of capillaries, reproduction of cells, and permeation of a liquid medicine is deteriorated. However, when the force that is applied to the scalp is too large, the user may feel inconvenience in use of the stamp-type apparatus, so there is a problem that a satisfactory hair care and treatment effects cannot be achieved.

### Documents of Related Art

(Patent Document 1) Korean Patent No. 10-1993706 (June 21, 2019)
(Patent Document 2) Korean Patent No. 10-1994345 (June 24, 2019)

### SUMMARY OF THE INVENTION

The present disclosure has been made in an effort to solve the general problems in the related art and an objective of the present disclosure is to provide a device for automatically operating a stamp-type fluid injector having fine needles for hair loss care, the device: making fine injuries by tapping the fine needles of the fluid injector on the scalp so that hair loss is cared for and treated well by production of capillaries, reproduction of cells, and permeation of a liquid medicine even into hair roots during recovery of the injuries; having the fluid injector simply mounted and separated; and being able to continuously and uniformly tap the scalp while automatically moving up and down the fluid injector to be conveniently used.

A device for automatically operating a stamp-type fluid injector having fine needles for hair loss care and treatment of the present disclosure includes: an outer case; a cover coupled to a lower portion of the inner case and having a hole at a center thereof; an inner housing fitted at a center in the outer case; a motor disposed over the inner housing; a coupler inserted in the inner housing to be able to move up and down therein and configured to be coupled to a fluid injector; and an actuator configured such that an eccentric portion of a cam fitted on a motor shaft of a motor and an upper portion of the coupler are fitted to upper and lower portions of a link so that the coupler is moved up and down in an operation hole of the inner housing by rotation of the motor. The device cares and treats hair loss by uniformly and continuously tapping the scalp by automatically moving up and down the fluid injector having fine protruding needles by operating the motor with the fluid injector inserted and coupled to the coupler through the hole of the cover.

Preferably, the coupler includes an outer cap inserted to be able to move up and down in an operation hole formed at a center of a lower portion of the inner housing, an elastic spring inserted in the outer cap, an inner cap fitted in the outer cap and elastically absorbing shock upward by the elastic spring, and a flexible cap fitted in the inner cap and receiving the fluid injector, wherein an upper portion of the outer cap is connected to the link of the actuator.

According to the present disclosure, the flexible cap may have ribs protruding at equal angles on an inner circumferential portion to hold the liquid injector and may have a top coupling protrusion at a center of a top thereof and a side coupling protrusion on an outer side of the top such that the protrusions are inserted in coupling holes formed at a center and an outer side of the inner cap.

According to the present disclosure, a ring-shaped protrusion may be formed on an outer circumferential portion of a lower portion of the flexible cap to seal an inner circumferential portion of the hole of the cover.

According to the present disclosure, tension parts having inward protrusions may be formed at equal angles on outer circumferential portion of a lower portion of the outer cap, locking protrusions locking the inward protrusions of the tension parts to prevent separation of the inward protrusion may be formed on an outer circumferential portion of an upper portion of the inner cap, and guide grooves guiding the locking protrusions moving up and down when the inner cap moves up and down may be formed under the locking protrusions.

According to the present disclosure, a substrate having one or more LEDs may be disposed inside the cover so that light from the LEDs is radiated to a scalp.

According to the present disclosure, a supporting plate having several supporting protrusions may be disposed under the cover and coupled to the outer case by a coupling ring.

According to the present disclosure, a hole is formed at the center of a cover coupled to the lower portion of an outer case such that an outer cap is moved up and down in the hole by a motor. An inner cap elastically operated by a spring is disposed under the outer cap, a flexible cap is disposed in the inner cap, and a fluid injector is simply inserted into and separated out of the flexible cap through the hole of the cover. Accordingly, it is possible to uniformly and continuously tap the scalp by automatically moving up and down a fluid injector by operating the motor. Further, a fluid injector can be more conveniently used, as compared with when it is manually used.

Accordingly, the fine needles of the fluid injector make injuries on the scalp; and capillaries are produced, cells are reproduced, and the liquid medicine smoothly permeates into the hair roots in the process of recovery of injuries, whereby an excellent effect of caring for and treating the hairs is achieved.

In particular, when the fluid injector taps the fine needles on the scalp, the inner cap combined with the fluid injector absorbs shock in the outer cap by the elastic spring, whereby it is possible to achieve softer feeling that is not stimulating in use. Accordingly, it is possible to achieve not only a hair care and treatment, but also high satisfaction in use.

### BRIEF DESCRIPTION OF THE DRAWINGS

The above and other objectives, features and other advantages of the present invention will be more clearly understood from the following detailed description when taken in conjunction with the accompanying drawings, in which:
FIG. 1 is a perspective view showing the entire external appearance of the present disclosure;
FIG. 2 is a perspective view with a cradle separated from FIG. 1;
FIG. 3 is a perspective bottom view of FIG. 1 without the cradle;
FIG. 4 is a cross-sectional perspective view of FIG. 3;
FIG. 5 is an exploded perspective view of FIG. 3;
FIG. 6 is an exploded cross-sectional perspective view showing an actuator, an outer cap, an inner cap, and a flexible cap of the present disclosure;
FIG. 7 is a cross-sectional view of side of FIG. 3;
FIG. 8 is a cross-sectional view of another side of FIG. 3;
FIG. 9 is a cross-sectional view showing an example of using the device of the present disclosure for the scalp;
FIGS. 10 and 11 are cross-sectional view showing main parts when the fluid injector is operated to move up and down by the structure shown in FIG. 6;
FIG. 12 is a perspective view showing a fluid injector that has been filled and registered in the related art; and
FIG. 13 is a cross-sectional view showing a structure for opening and closing a nozzle of FIG. 12.

### DETAILED DESCRIPTION OF THE INVENTION

Hereinafter, exemplary embodiments of the present disclosure will be described in detail with reference to the accompanying drawings.

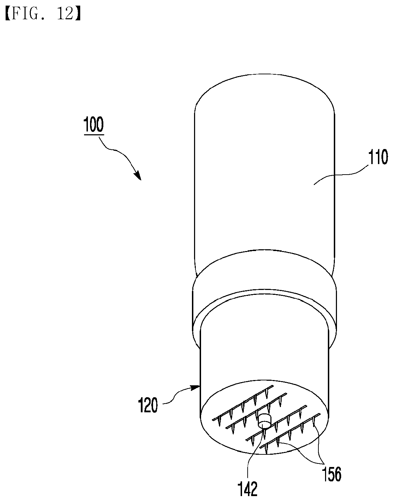

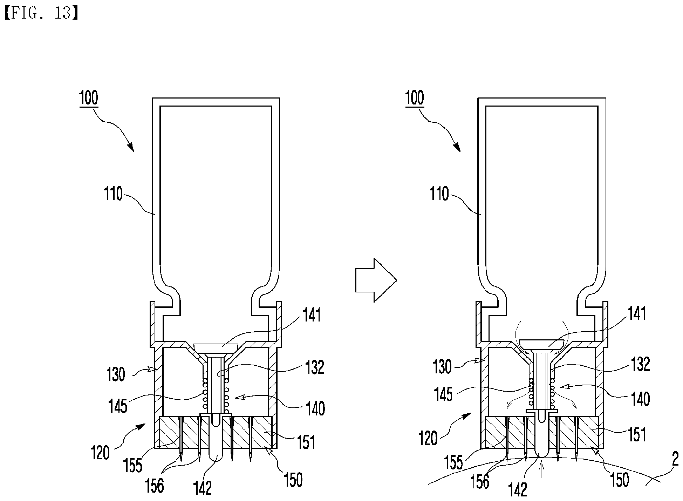

A device for automatically operating a stamp-type fluid injector having fine needles for hair loss care of the present disclosure is configured to be mounted on a fluid injector 200 shown in FIGS. 12 and 13 to automatically operate the fluid injector 200.

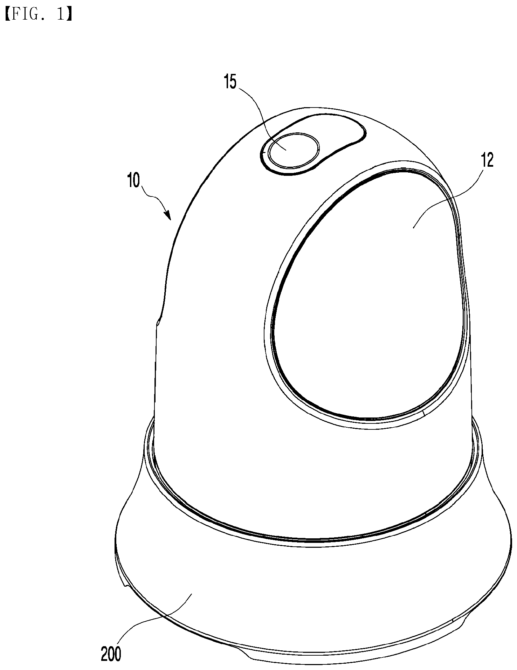

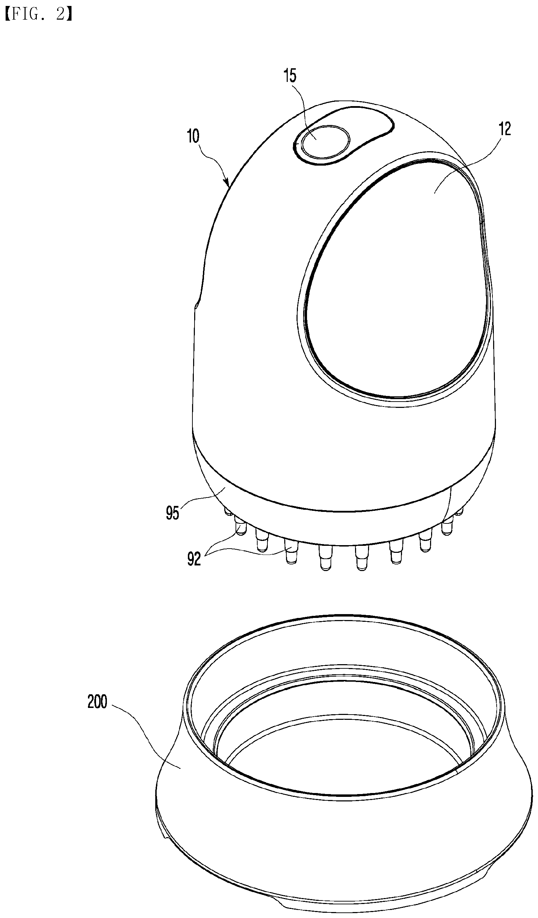

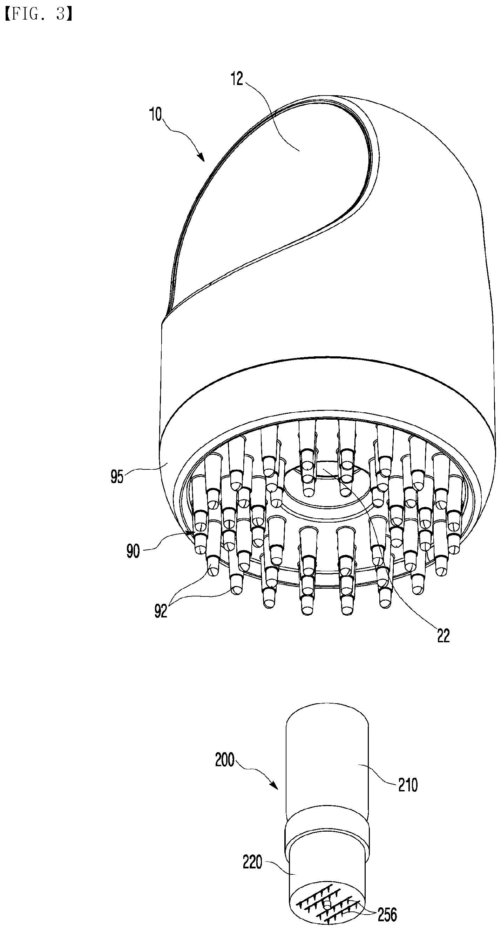

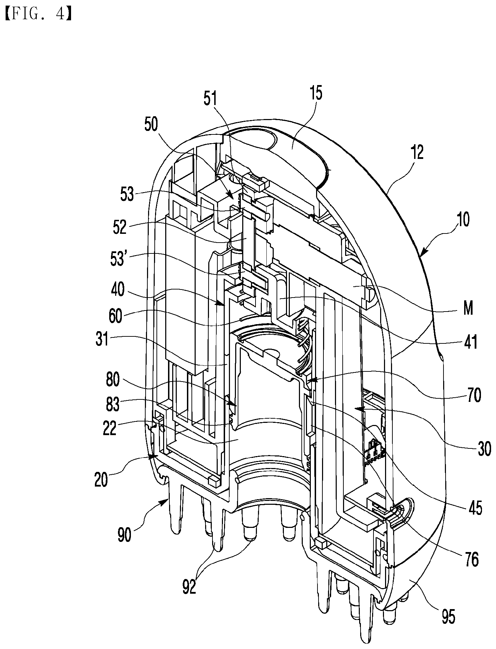

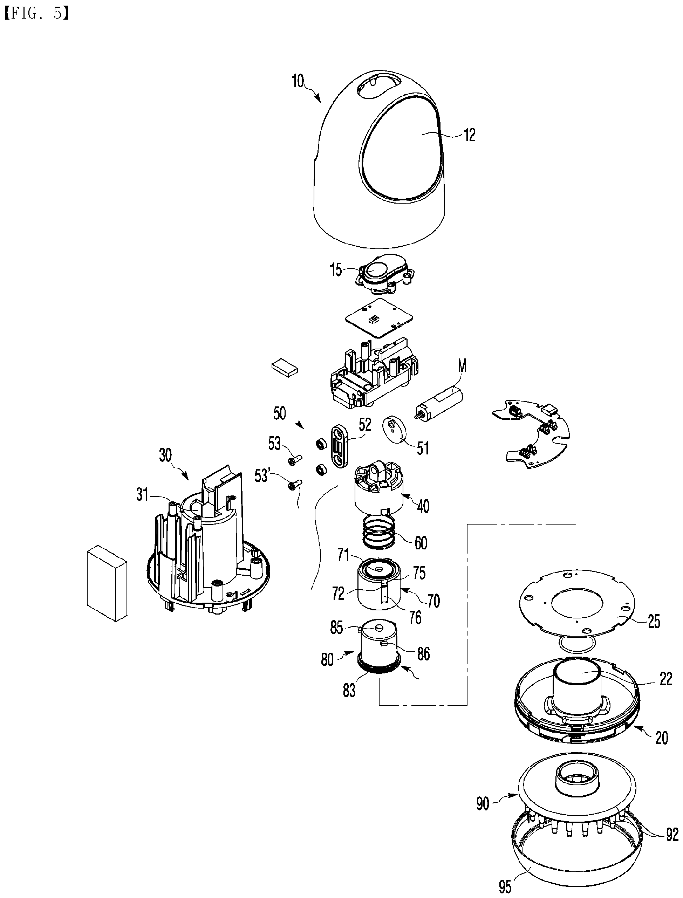

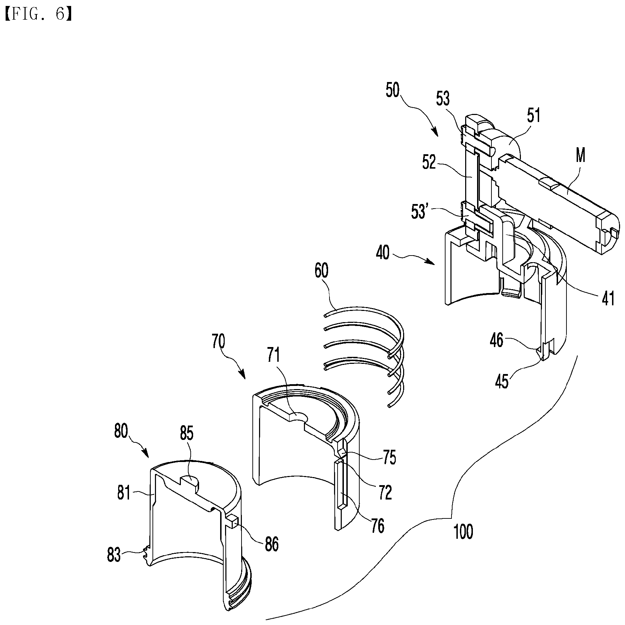

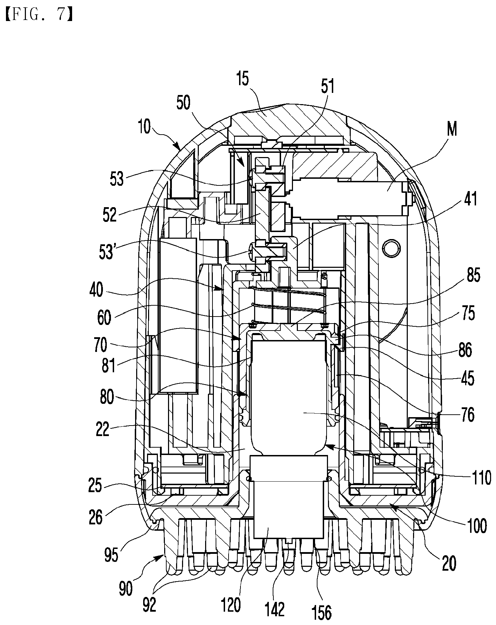

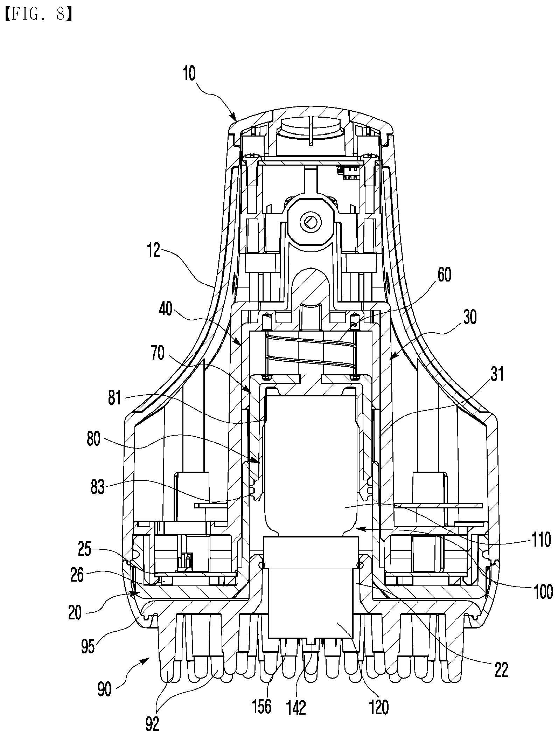

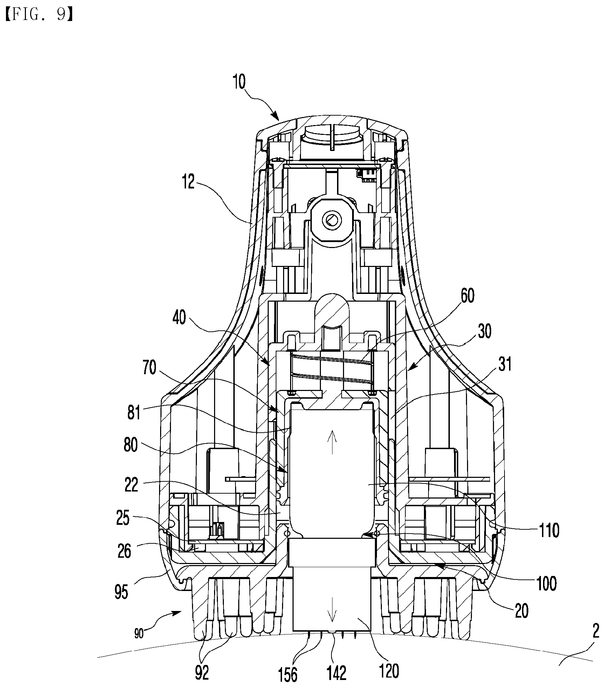

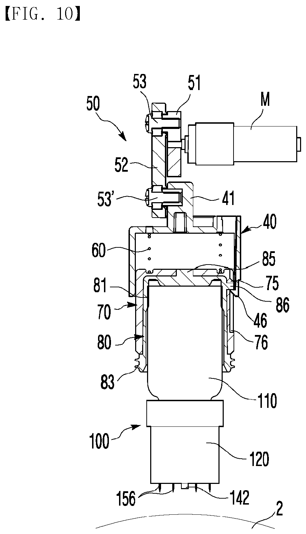

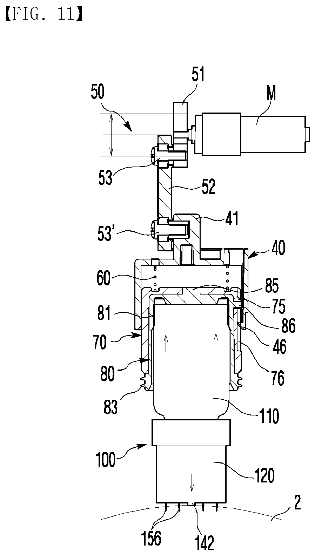

That is, the present disclosure, as shown in FIGS. 1 to 11, includes an outer case 10, a cover 20, an inner housing 30, a coupler 100, and an actuator 50.

The outer case 10 has a circular shape with an open bottom and grips 12 recessed on both sides of the upper portion thereof to be held by a hand, and an operation switch 15 is disposed over the grips.

The cover 20 is coupled to the outer case 10 to cover the lower portion of the outer case 10 and a hole 22 in which a fluid injector 220 can be inserted is formed at the center of the cover.

A substrate 25 having one or more LEDs 26 thereon is disposed inside the cover 20 and light from the LEDs are radiated to the scalp through the cover, whereby the wavelengths of the light from the LEDs stimulate the scalp to increase the hair care and treatment effect. Since the wavelengths of the light from the LEDs 26 are different in accordance with the colors, the LEDs 26 may be configured such that one color or several colors (red, yellow, green, and blue) may be sequentially turned on and off.

A supporting plate 90 having several supporting protrusions 92 is disposed under the cover 20 and is coupled to the outer case 10 by a coupling ring 95. The supporting protrusions 92 have a function of tapping the scalp and a function of securing a distance from a scalp so that fine needles of the fluid injector 200 can tap the scalp.

The coupler 100 is inserted in the inner housing 30 to be able to move up and down therein and configured to be coupled to the fluid injector 200.

The actuator 50 is configured such that an eccentric portion of a cam 51 fitted on a motor shaft of a motor M and the upper portion of the coupler are coupled to the upper and lower portions of a link 52 by shaft pins 53 and 53' so that the coupler is moved up and down in an operation hole 31 of the inner housing by rotation of the motor.

The coupler 100 includes an outer cap 40, an elastic spring 60, an inner cap 70, and a flexible cap 80.

The outer cap 40 has a structure with an open bottom and is inserted in the inner housing 30 to move up and down in the operation hole 31.

The elastic spring 60 is inserted in the outer cap 40 and the inner cap 70 is fitted on the lower portion of the elastic spring to elastically upwardly absorb shock.

The inner cap 70 has a locking protrusion 75 formed on the upper circumferential portion and a guide groove 76 vertically formed under the locking protrusion. The outer cap 40 has a tension part 45 having an inward protrusion 46 on the lower circumferential portion. The locking protrusion 75 of the inner cap is elastically fitted to the inward protrusion 46 of the tension part 45, whereby when the inner cap moves up and down, the locking protrusion is guided up and down in the guide groove 76.

The flexible cap 80 is made of flexible silicon or flexible resin and is disposed in the inner cap 70. The flexible cap 80 is configured such that a liquid medicine capsule 210 of the fluid injector 200 is inserted and mounted therein.

The flexible cap 80 has ribs 81 protruding at equal angles on the inner circumferential portion to hold the liquid medicine capsule of a liquid injector and has a top coupling protrusion 85 at the center of the top and a side coupling protrusion 86 on the outer side of the top such that the protrusions are inserted in coupling holes 71 and 72 formed at the center and the outer side of the inner cap 70.

A ring-shaped contact protrusion 83 is formed on the outer circumferential portion of the lower portion of the flexible cap 80 to seal the inner circumferential portion of the hole 22.

That is, when the outer cap 40, the inner cap 70, and the flexible cap 80 are moved up and down in the operation hole 31 of the inner housing by the motor, the fluid injector 209 taps fine needles on a scalp while moving up and down, thereby automatically making fine injuries and supplying a liquid medicine. Further, the inner cap 70 and the flexible cap 80 absorb shock, which is generated by fine needles tapping the scalp, while compressing the elastic spring 60, thereby smoothly tapping the scalp with little shock.

The fluid injector 200, which is well known in the art, as shown in FIGS. 12 and 13, is combined with an applicator 220 at the inlet of the liquid medicine capsule 210 containing a liquid medicine, and includes a connection housing 230, a cap 240, and a needle assembly 250. The connection housing 230 is coupled to the liquid medicine capsule and has a nozzle hole 232 having a tapered upper end at the center. The needle assembly 250 is coupled to the lower portion of the connection housing and has several needle plates 255 having fine needles 256 protruding from a body 251. The cap 240 has a top pin fitted in the nozzle hole 232 of the connection housing and a bottom pin 242 protruding downward from the needle assembly, in which the top pin 241 and the bottom pin 242 are coupled up and down and inserted in a spring 245 to elastically move up and down. When a user taps the fluid injector 200 on the scalp, the fine needles 256 of the needle assembly make fine injuries on the scalp. Further, capillaries are produced in the process of recovering from the injuries, so blood circulation is improved and the liquid medicine effectively permeates even to the hair roots with reproduction of cells, whereby the effect of hair loss care and treatment is considerably increased.

It should be noted that the detailed structure of the liquid injector 200 may be changed and any fluid injector can be applied to the device of the present disclosure as long as it has a structure that supplies a liquid medicine while making fine injuries with fine needles when it is tapped on the scalp.

Unstated reference numeral, '2' indicates the scalp and the numeral '150' indicates a cradle.

The operation and function of the present disclosure having the configuration described above are described hereafter.

First, the fluid injector 200 shown in FIGS. 12 and 13 are prepared.

That is, the applicator 220 having the protruding fine needles 256 is coupled to the liquid medicine capsule 210.

The prepared liquid injector 200 is inserted into the hole 22 formed at the center of the cover 20 from under the outer case 10.

In this process, the liquid medicine capsule 210 of the liquid injector is inserted into the flexible cap 80 fitted in the inner cap 70.

The flexible cap 80 is made of a flexible material, thereby enabling the liquid medicine capsule 210 of the fluid injector to be simply coupled by forcible fitting and to be simply separated by forcible pulling after the liquid medicine is fully used.

In particular, since the ribs 81 protruding from the inner circumferential portion of the flexible cap 80 and hold the outer circumferential portion of the liquid medicine capsule 210 using their own elasticity, so the fluid injector can be more firmly coupled.

As described above, the fluid injector 200 is coupled to the device of the present disclosure and then the operation switch 15 coupled to the top of the outer case is pressed to turn on.

When the operation switch 15 is turned on, the motor M is operated and the outer cap 40 is moved up and down in the operation hole 31 of the inner housing 30 by the actuator 50.

That is, since the eccentric portion of a cam 51 fitted on the motor shaft of the motor M and the connection portion 41 at the upper portion of the outer cap 40 are coupled to the upper and lower portions of the link 52 by the shaft pins 53 and 53', when the cam 51 is rotated by the motor, the link 52 operates like a cam, thereby moving up and down the outer cap 40 in the operation hole 31 of the inner housing 30.

As the outer cap 40 is moved up and down, the inner cap 70 fitted in the outer cap and the flexible cap 80 fitted in the inner cap are moved up and down together, thereby moving up and down the fluid injector 200.

In this process, the ring-shaped contact protrusion 83 protruding from the outer circumferential portion of the flexible cap 80 keeps sealing while moving up and down in close contact with the inner surface of the hole 22 of the cover.

With the power on, as described above, a user holds the grips 12 on the upper portion of the outer case and moves the lower portion of the outer case to the position of the scalp to be supplied with the liquid medicine.

The supporting protrusions 92 of the supporting plate 90 coupled to the bottom of the cover 20 are disposed under the outer case 10 to be supported on the scalp for massaging the scalp and to maintain a predetermined distance from the scalp.

In this state, the fluid injector 200 is moved up and down at the center in the lower portion of the outer housing 10. In this process, the fine needles 256 of the needle assembly 250 make fine injuries while tapping the scalp and capillaries are produced in the process of recovering from the injuries. Accordingly, blood circulation is improved at the hair roots and reproduction of cells is promoted.

Further, as the cap 240 of the fluid injector 200 comes in contact with the scalp, the nozzle hole 232 of the connection housing 230 is opened, so the liquid medicine is supplied to the scalp and permeates even to the hair roots.

That is, the fine needles 256 of the fluid injector make injuries on the scalp; and capillaries are produced, cells are reproduced, and the liquid medicine smoothly permeates into the hair roots in the process of recovery of injuries, whereby an excellent effect of not only caring, but treating the hairs is achieved.

In particular, the flexible cap 80 and the inner cap 70 combined with the fluid injector 200 absorb shock, which is generated by the fine needles tapping the scalp, while compressing the elastic spring 60 in the outer cap 40.

In this process, the inward protrusions 46 of the tension parts 45 formed at equal angles on the outer circumferential portion of the lower portion of the outer cap 40 are guided to move up and down in the guide grooves 76 of the inner cap and the inner cap absorbs shock while compressing the elastic spring 60, so the shock that is applied to the scalp is absorbed. Accordingly, the user can use the device while softly tapping the scalp without inconvenience.

When the inner cap 70 is returned downward in the outer cap 40 by the elasticity of the elastic spring 60, the locking protrusions 75 of the inner cap are locked to the inward protrusions 46 of the tension parts 45 formed at equal angles on the outer circumferential portion of the lower portion of the outer cap 40 to be supported without separating.

As described above, the fine needles make fine injuries on the scalp while the fluid injector 200 automatically moves up and down; capillaries are produced, cells are reproduced, and the liquid medicine smoothly permeates into the hair roots in the process of recovering from the injuries; and this process is continuously performed. Accordingly, the device is simply used and increases the hair care and treatment effect in comparison to the manual work of the related art.

Further, since the substrate 25 having LEDs 26 having one or more colors (red, yellow, green, and blue) is disposed inside the cover 20 such that light from the LEDs is simultaneously or sequentially radiated to the scalp through the cover, the wavelengths of the light from the LEDs variously stimulate the scalp. Accordingly, the hair care and treatment effect is further increased.

## Claims

1. A device for automatically operating a stamp-type fluid injector having fine needles for hair loss care and treatment, the device comprising:
an outer case;
a cover coupled to a lower portion of the inner case and having a hole at a center thereof;
an inner housing fitted in the outer case;
a motor disposed over the inner housing;
a coupler inserted in the inner housing to be able to move up and down therein and configured to be coupled to a fluid injector; and
an actuator configured such that an eccentric portion of a cam fitted on a motor shaft of a motor and an upper portion of the coupler are fitted to upper and lower portions of a link so that the coupler is moved up and down in an operation hole of the inner housing by rotation of the motor,
wherein the device cares and treats hair loss by uniformly and continuously tapping the scalp by automatically moving up and down the fluid injector having fine protruding needles by operating the motor with the fluid injector inserted and coupled to the coupler through the hole of the cover.

2. The device of claim 1, wherein the coupler includes an outer cap inserted to be able to move up and down in an operation hole formed at a center of a lower portion of the inner housing, an elastic spring inserted in the outer cap, an inner cap fitted in the outer cap and elastically absorbing shock upward by the elastic spring, and a flexible cap fitted in the inner cap and receiving the fluid injector, wherein an upper portion of the outer cap is connected to the link of the actuator.

3. The device of claim 2, wherein the flexible cap has ribs protruding at equal angles on an inner circumferential portion to hold the liquid injector and has a top coupling protrusion at a center of a top thereof and a side coupling protrusion on an outer side of the top such that the protrusions are inserted in coupling holes formed at a center and an outer side of the inner cap.

4. The device of claim 2, wherein tension parts having inward protrusions are formed at equal angles on outer circumferential portion of a lower portion of the outer cap, locking protrusions locking the inward protrusions of the tension parts to prevent separation of the inward protrusion are formed on an outer circumferential portion of an upper portion of the inner cap, and guide grooves guiding the locking protrusions moving up and down when the inner cap moves up and down are formed under the locking protrusions.

5. The device of claim 1 or 2, wherein a substrate having one or more LEDs is disposed inside the cover so that light from the LEDs is radiated to the scalp.

6. The device of claim 1 or 2, wherein a supporting plate having several supporting protrusions is disposed under the cover and coupled to the outer case by a coupling ring.
